(19) Europäisches Patentamt / European Patent Office / Office européen des brevets

(11) **EP 3 484 702 B2**

(12) **NOUVEAU FASCICULE DE BREVET EUROPEEN**
Après la procédure d'opposition

(45) Date de publication et mention de la décision concernant l'opposition:
**02.04.2025 Bulletin 2025/14**

(45) Mention de la délivrance du brevet:
**16.06.2021 Bulletin 2021/24**

(21) Numéro de dépôt: 17748830.1

(22) Date de dépôt: **11.07.2017**

(51) Classification Internationale des Brevets (IPC):
*B32B 5/02* (2006.01)   *B32B 5/26* (2006.01)
*B32B 7/04* (2019.01)   *B32B 27/12* (2006.01)
*B32B 7/14* (2006.01)   *B32B 27/30* (2006.01)
*B32B 27/32* (2006.01)

(52) Classification Coopérative des Brevets (CPC):
**B32B 5/022; A61F 13/15699; B32B 5/26; B32B 7/05; B32B 7/14; B32B 27/12; B32B 27/302; B32B 27/327;** B32B 2262/0253; B32B 2307/51; B32B 2307/54; B32B 2555/00; B32B 2555/02

(86) Numéro de dépôt international:
**PCT/FR2017/051899**

(87) Numéro de publication internationale:
**WO 2018/011516 (18.01.2018 Gazette 2018/03)**

(54) **ENSEMBLE LAMINE ET PROCÉDÉ DE FABRICATION**

LAMINAT UND HERSTELLUNGSVERFAHREN

LAMINATE AND ITS METHOD OF MANUFACTURING

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **15.07.2016 FR 1656808**

(43) Date de publication de la demande:
**22.05.2019 Bulletin 2019/21**

(73) Titulaire: **Aplix**
**44850 Le Cellier (FR)**

(72) Inventeurs:
• **MOINARD, Nathalie**
**44850 Le Cellier (FR)**
• **MARCHE, Thierry**
**44850 Le Cellier (FR)**

(74) Mandataire: **Cabinet Beau de Loménie**
**158, rue de l'Université**
**75340 Paris Cedex 07 (FR)**

(56) Documents cités:
**EP-A1- 0 803 602     EP-A1- 2 799 606
EP-B1- 3 316 834     WO-A1-03/007864
WO-A1-2005/065947     WO-A1-2005/110748
WO-A1-2006/073975     WO-A1-2007/061486
WO-A1-2014/159724     WO-A1-94/20298
DE-U1- 29 825 018     US-A- 6 114 263
US-A1- 2006 148 354     US-B1- 6 383 431**

EP 3 484 702 B2

**Description**

Arrière-plan de l'invention

Le présent exposé concerne des ensembles laminés comprenant des nappes de non-tissé.

Le présent exposé concerne, plus particulièrement, des nappes de non-tissé et des ensembles laminés pouvant être utilisés dans le domaine de l'hygiène, notamment pour la fabrication d'oreilles élastiques de couches culottes.

Ces ensembles laminés comprennent généralement deux nappes de non-tissé solidaires d'un film élastique interposé entre les deux nappes de non-tissé, les deux nappes de non-tissé et le film élastique étant assemblés par laminage pour former un ensemble laminé, aussi appelé trilaminé.

Ces nappes de non-tissé présentent généralement une capacité d'élongation inférieure à la capacité d'élongation du film élastique si bien qu'il est difficile d'obtenir aisément un produit avec une capacité d'élongation désirée. Il est alors nécessaire de réaliser un traitement sur le trilaminé pour l'étirer transversalement afin de lui donner une capacité d'élongation transversale désirée.

Cependant, lors de la fabrication d'un tel ensemble laminé, lors de ce traitement, l'apparition de certains défauts, tels que des trous dans l'ensemble laminé, plus particulièrement dans le film élastique, peuvent entraîner la rupture de l'ensemble laminé sur la ligne de fabrication ou lorsque l'on tire dessus dans la direction d'allongement ou encore des pollutions non désirées de l'ensemble laminé entraînant des arrêts de la ligne de production afin de la nettoyer. Par ailleurs, l'ensemble laminé comprenant des trous, des coupures, des fentes ou qui est pollué ne peut être utilisé et il est mis au rebut. Certains trous sont visibles à l'oeil nu lorsque l'ensemble laminé est à l'état non étiré et peuvent s'agrandir lorsque le laminé est étiré. Il est alors aisé de les détecter. En revanche, d'autres trous peuvent être présents dans l'élastique seul et être visibles à l'œil nu uniquement lorsque l'ensemble laminé est à l'état étiré. Il est alors difficile de les détecter. D'autres trous, coupures ou fentes peuvent également se former, d'une part, lors du traitement décrit précédemment, et/ou, d'autre part, lors de la première extension de l'ensemble laminé, par exemple lorsque le laminé est utilisé sur le produit fini, telle qu'une couche culotte entraînant sa rupture. Ces trous, coupures et/ou fentes sont alors très difficiles à détecter puisque détectés sur l'application finale. Dans tous ces cas, ces trous dégradent la qualité perçue par l'utilisateur/l'usager du produit fini. Les fentes dans le film élastique sont d'autant plus difficiles à détecter qu'elles s'étendent selon la direction longitudinale et peuvent se former notamment lors du traitement décrit précédemment en raison du fort étirement transversal donnant au trilaminé une capacité d'élongation transversale désirée et/ou notamment en raison de la présence de points durs inclus dans le film élastique. Le document EP0803602 divulgue un corps élastique composite.

Objet et résumé de l'invention

Le présent exposé vise à remédier au moins en partie à ces inconvénients.

A cet effet, le présent exposé concerne un ensemble laminé tel que défini par la revendication 1 ou la revendication 2, s'étendant selon une direction longitudinale et une direction latérale, orthogonale à la direction longitudinale, l'ensemble comprenant une nappe de non-tissé et un film élastique laminés ensemble, la nappe de non-tissé comprenant au moins une zone activée s'étendant sur une longueur de la nappe de non-tissé mesurée dans la direction longitudinale et sur une largeur strictement inférieure à une largeur de la nappe de non-tissé mesurée dans la direction latérale, le taux d'activation de la zone activée de la nappe de non-tissé dans la direction latérale étant différent du taux d'activation du film élastique dans la direction latérale, le taux d'activation de la zone activée de la nappe de non-tissé dans la direction latérale étant compris entre 20 et 200 %.

La nappe de non-tissé et le film élastique présentent des taux d'activation différents, on comprend que la nappe de non-tissé est activée avant d'être laminée avec le film élastique.

Grâce à cette activation de la nappe de non-tissé avant assemblage avec le film élastique, on obtient un ensemble laminé qui présente une bonne capacité d'élongation dans la direction latérale et il est possible de réduire, voire de supprimer, l'activation de l'ensemble laminé, c'est-à-dire de réduire le taux d'activation de l'ensemble laminé, voire que le taux d'activation de l'ensemble laminé soit égal à zéro (pas d'activation de l'ensemble laminé).

Dans le présent exposé, le terme non-tissé englobe tous les non-tissés tombant dans la définition communément admise par l'homme de l'art, typiquement une nappe comprenant des fibres et/ou de filaments qui sont consolidé(e)s.

On entend par film élastique, un film qui peut être étiré sans se rompre sous l'effet d'une force d'étirement exercée dans la direction latérale et pouvant reprendre sensiblement sa forme et ses dimensions initiales après relâchement de cette force d'étirement. Il s'agit par exemple d'un film qui conserve une déformation résiduelle ou rémanence après élongation et relâchement (déformation résiduelle aussi appelée « permanent set » ou « SET ») inférieure à 20 %, plus préférentiellement inférieure à 5 %, de sa dimension initiale (avant élongation) pour un allongement de 100 % de sa dimension initiale, à température ambiante (23°C).

On entend par activation un procédé au cours duquel, l'ensemble laminé passe dans un module d'activation qui étire l'ensemble laminé dans une direction perpendiculaire au sens de défilement de l'ensemble laminé dans le module d'activation afin d'augmenter la capacité d'élongation des nappes de non-tissé de l'ensemble laminé.

L'activation de l'ensemble laminé est un traitement permettant de réduire la cohésion de la structure de la nappe de non-tissé, et d'assurer que l'ensemble laminé puisse être étiré aisément (avec un moindre effort, par exemple à 10N).

Les inventeurs ont identifié qu'une des sources de défaut et/ou de pollution et/ou de formation de trous, de coupures et/ou de fentes trouvait son origine dans le passage de l'ensemble laminé dans le module d'activation avec des taux d'activation élevés, par exemple supérieurs à 200 %.

Ainsi, les inventeurs ont identifié que l'activation de l'ensemble laminé étant réduite, voire supprimée, les défauts et/ou les pollutions et/ou les trous de l'ensemble laminé liés au passage de l'ensemble laminé dans un module d'activation étaient réduits, voire supprimés.

En effet, le module d'activation comprend deux rouleaux d'activation munis chacun d'un empilement de disques parallèles espacés les uns des autres. Les rouleaux d'activation et les disques comprennent chacun un axe de symétrie qui est orienté selon la direction latérale. Les disques du premier rouleau d'activation sont disposés dans les espaces inter-disques du deuxième rouleau d'activation (préférentiellement au milieu des espaces inter-disques) et les axes de symétrie des deux rouleaux d'activation sont espacés l'un de l'autre d'une distance inférieure au diamètre des disques de sorte que les zones de l'ensemble laminé passant entre les disques des rouleaux sont étirées dans la direction latérale, formant les zones activées. A ces endroits, les fibres et/ou filaments de la nappe de non-tissé sont étiré(e)s et/ou cassé(e)s et le film élastique est étiré. On comprend que plus la distance entre les axes de symétrie des deux rouleaux d'activation est faible, plus l'interpénétration des disques du premier rouleau par rapport au deuxième rouleau est grande et plus le taux d'activation est important.

Le taux d'activation est le rapport de la différence entre la distance entre l'extrémité de deux disques adjacents appartenant chacun à un rouleau d'activation différent et la distance entre l'extrémité de ces deux mêmes disques projetée sur l'axe de symétrie d'un rouleau d'activation rapportée à cette distance entre l'extrémité de ces deux mêmes disques projetée sur l'axe de symétrie d'un rouleau d'activation exprimé en pourcent.

Lorsque le taux d'activation est important, l'étirement de l'ensemble laminé est important et les risques de formation de trous, de coupures ou de fentes dans l'ensemble laminé et en particulier dans le film élastique augmentent.

Par ailleurs, lorsque la nappe de non-tissé est collée sur le film élastique, les forces exercées sur l'ensemble laminé lors de son passage dans le module d'activation augmentent et les disques pénétrant de manière plus importante dans l'ensemble laminé, les disques peuvent être en contact avec la colle qui solidarise la nappe de non-tissé et le film élastique. Ainsi, le (ou les) disque(s) d'activation étant en contact avec la colle, l'ensemble laminé reste collé au(x) disque(s) d'activation, et, compte-tenu de la vitesse de la ligne, en quelques secondes le produit s'accumule dans le bloc d'activation et la ligne de fabrication doit être arrêtée en urgence au risque de casser un ou plusieurs disques d'activation. Une telle casse nécessiterait une intervention longue et lourde de maintenance. En l'absence de casse, il est tout de même nécessaire de nettoyer le module d'activation et de redémarrer la ligne de fabrication. Les disques d'activation ont encore plus de risque d'être contaminés par de la colle lorsque les ensembles laminés présentent une nappe de non-tissé avec un faible grammage. Dans certains cas, cette colle peut se déposer sur l'ensemble laminé et le polluer. Cette colle peut également arracher des fibres à la nappe de non-tissé qui peuvent ensuite se déposer sur l'ensemble laminé et le polluer. Par ailleurs, des fibres étant arrachées, il peut se former un trou non désiré dans la nappe de non-tissé.

La nappe de non-tissé étant déjà activée, une fois la nappe de non-tissé laminée avec le film élastique, l'ensemble laminé présente une capacité d'élongation satisfaisante et l'ensemble laminé peut ne pas être activé. Le film élastique n'est donc pas activé. On évite ainsi avantageusement les risques de contamination liés au transfert de colle sur les disques du module d'activation nécessitant notamment l'arrêt en urgence de la ligne de fabrication, on évite d'étirer le film élastique et on évite de former des trous, des coupures et/ou des fentes dans le film élastique. On peut ainsi réduire le taux de rebut de l'ensemble laminé et également réduire les temps d'arrêt de la ligne de production afin de la nettoyer.

On peut également envisager que la zone activée de la nappe de non-tissé ait une largeur au moins égale à la largeur du film élastique.

Afin d'éviter des problèmes de délamination lorsque l'on active un ensemble laminé, la largeur de la zone d'activation de l'ensemble laminé est inférieure à la largeur du film élastique.

La nappe de non-tissé ayant été activée avant son assemblage avec le film élastique, il est possible d'activer cette nappe de non-tissé sur une largeur qui peut être égale ou supérieure à la largeur du film élastique. On comprend que bien entendu la largeur de la zone activée peut être inférieure à la largeur du film élastique.

Le taux d'activation de la zone activée de la nappe de non-tissé dans la direction latérale peut être compris entre 40 et 200 %, plus particulièrement entre 40 et 160 %.

Par ailleurs, l'ensemble laminé comprenant une nappe de non-tissé qui a été activée avant son assemblage avec le film élastique peut être activé. Toutefois, comme la nappe de non-tissé a été activée avant son assemblage avec le film élastique, le taux d'activation de l'ensemble laminé peut être moins important que si la nappe de non-tissé n'avait pas été activée avant son assemblage avec le film élastique, à capacité d'élongation identique de l'ensemble laminé. On réduit ainsi avantageusement les risques de contamination liés au transfert de colle sur les disques du module d'activation nécessitant notamment l'arrêt en urgence de la ligne de fabrication, on réduit l'étirement du film élastique et on réduit le risque de formation de trous, de coupures et/ou de fentes dans le film élastique. Le transfert de colle aux disques est d'autant plus important que les nappes de non-tissé utilisées présentent un faible grammage. On peut ainsi réduire le taux de rebut de l'ensemble laminé et également réduire les temps d'arrêt de la ligne de production afin de la nettoyer.

Par exemple, l'ensemble laminé peut être activé à moins de 200 % dans la direction latérale, de préférence à moins de 150 %, encore plus de préférence à moins de 125%, en particulier à moins de 100 %.

Le film élastique peut présenter une largeur inférieure à la largeur de la nappe de non-tissé.

Le film élastique peut être co-extrudé avec un matériau non-élastique afin de former un film dont la largeur est sensiblement égale à la largeur de la nappe de non-tissé, le film élastique étant assemblé à la zone activée de la nappe de non-tissé.

Le film élastique peut être formé par une colle élastique.

Le film de colle élastique peut alors être extrudé sur la nappe de non-tissé et ensuite laminé avec cette nappe de non-tissé. La zone activée peut s'étendre sur toute la longueur de la nappe de non-tissé mesurée dans la direction longitudinale.

Une nappe de non-tissé est par exemple constituée d'une nappe de fibres et/ou filaments issue de la technologie Dry-laid (voie sèche), Wet-laid (voie humide), ou Spun-laid (voie fondue/extrudée) et consolidés par liaison mécanique, thermique, chimique et/ou adhésive.

La nappe de non-tissé peut être un non-tissé cardé calandré.

Le non-tissé cardé calandré est un non-tissé comprenant une nappe de fibres présentant des points de consolidations de la nappe répartis de manière sensiblement homogène sur toute la nappe par une consolidation thermique. La consolidation assure une certaine cohésion des fibres permettant leur manipulation et leur transport, notamment leur enroulement sous forme de bobine et leur déroulement. L'activation de la nappe de non-tissé cardé calandré permet d'allonger et/ou de casser les fibres de la nappe de non-tissé et/ou de déformer les points de consolidation de la nappe. On augmente ainsi la capacité d'élongation de la nappe de non-tissé.

Les fibres de la nappe de non-tissé cardé calandré sont comprises entre 1 et 8 dTex (déciTex), de préférence entre 1,3 et 6,7 dTex, encore plus de préférence entre 1,6 et 5,5 dTex.

Le Tex est l'unité SI de finesse des fibres textiles. Elle exprime le poids en gramme de 1 000 m (mètre) de longueur des fibres.

La nappe de non-tissé est une première nappe de non-tissé, l'ensemble laminé comprend une deuxième nappe de non-tissé et le film élastique peut être interposé entre la première et la deuxième nappe de non-tissé, ou la deuxième nappe de non-tissé peut être au moins partiellement interposée entre la première nappe de non-tissé et le film élastique.

Le film élastique étant interposé entre la première et la deuxième nappe de non-tissé, seules les nappes de non-tissé sont accessibles à l'utilisateur.

Le film élastique peut être formé par une colle élastique.

La colle élastique peut être extrudée sur la première nappe de non-tissé et la première nappe de non-tissé enduite de la colle élastique peut être ensuite laminé avec la deuxième nappe de non-tissé ou inversement.

La colle élastique peut être également extrudée directement entre les deux nappes de non-tissé et ensuite, les deux nappes de non-tissé et la colle élastique sont laminés l'un avec l'autre.

Le film élastique peut être formé par une colle élastique et peut être co-extrudé avec un matériau non-élastique formé par une colle non élastique afin de former un film dont la largeur est sensiblement égale à la largeur de la nappe de non-tissé, le film élastique étant assemblé à la zone activée de la nappe de non-tissé.

_ Le film élastique peut ne pas être activé.

La deuxième nappe de non-tissé peut être dépourvue de zone activée. La deuxième nappe de non-tissé n'est donc pas activée.

La deuxième nappe de non-tissé comprend au moins une zone activée s'étendant sur une longueur de la nappe de non-tissé mesurée dans la direction longitudinale et sur une largeur strictement inférieure à une largeur de la nappe de non-tissé mesurée dans la direction latérale, le taux d'activation de la zone activée de la deuxième nappe de non-tissé dans la direction latérale pouvant être compris entre 20 et 200 %.

Le taux d'activation de la zone activée de la deuxième nappe de non-tissé dans la direction latérale peut être compris entre 20 et 200 %, en particulier entre 40 et 200 %, encore plus en particulier entre 40 et 160 %.

Grâce à l'activation séparée de la première et de la deuxième nappe de non-tissé, il est possible, en fonction de la nature de la première et de la deuxième nappe de non-tissé d'activer à des taux différents chaque nappe de non-tissé afin de s'adapter au mieux à la nature de chaque nappe de non-tissé pour obtenir des ensembles laminés satisfaisants.

Le taux d'activation de la zone activée de la deuxième nappe de non-tissé dans la direction latérale peut être différent du taux d'activation du film élastique dans la direction latérale.

On comprend que l'ensemble laminé peut être activé après laminage et assemblage des deux nappes de non-tissé et du film élastique. Cependant, la deuxième nappe de non-tissé qui a été activée avant son assemblage avec le film élastique présentera un taux d'activation différent du taux d'activation du film élastique.

Bien entendu, lorsque l'ensemble laminé n'est pas activé, le taux d'activation du film élastique étant égal à zéro, le taux d'activation de la deuxième nappe de non-tissé est différent du taux d'activation du film élastique.

La nappe de non-tissé peut être un non-tissé de type Spunlace.

Le non-tissé de type Spunlace est un non-tissé formé à partir de filaments (sans fin) et/ou de fibres d'une longueur généralement comprise entre 30 et 60 mm (millimètre), consolidé(e)s par hydro-liage. Le non-tissé de type Spunlace est utilisé couramment dans le domaine de l'hygiène pour sa douceur et sa capacité naturelle de déformation. Du fait de sa capacité naturelle de déformation, le non-tissé Spunlace est particulièrement sujet au phénomène de neckdown, c'est-à-dire que lorsqu'une tension lui est appliquée dans une direction donnée, il subit, dans la direction orthogonale, une déformation importante. Par conséquent, sur une ligne de production où elle est soumise à une forte tension longitudinale, une nappe en non-tissé de type Spunlace se rétrécit sensiblement dans la direction latérale. Ainsi, pour une largeur souhaitée de l'ensemble laminé, on prévoit généralement une nappe de non-tissé de type Spunlace de largeur supérieure afin de tenir compte du phénomène de neckdown.

Grâce à l'activation de la nappe de non-tissé de type Spunlace, on peut prévoir une nappe de non-tissé de type Spunlace présentant une largeur inférieure à la largeur de la nappe de non-tissé de type Spunlace qu'il aurait fallu prévoir si la nappe de non-tissé de type Spunlace n'avait pas été activée, à largeur d'ensemble laminé constante. On réduit ainsi les coûts de production de l'ensemble laminé. Il est alors possible en activant préalablement la nappe de non-tissé Spunlace d'ajuster sa largeur, supérieure et/ou égale, inférieure et/ou égale à la largeur du non-tissé Spunlace avant son passage sur la ligne de fabrication.

La nappe de non-tissé peut être un non-tissé de type Spunmelt.

Le non-tissé de type Spunmelt comprend les non-tissés de type Spunbond, les non-tissés de type Meltblown et les non-tissés comprenant au moins une couche de type Spunbond et au moins une couche de type Meltblown (par exemple, SM, SMS, SSMMS, SMSMS, SMMMS, SMMS, SSMMSS, SSMMMSS, SSMMMSSS,... ou tout autre combinaison de couches S et M, S représentant une couche de type Spunbond et M représentant une couche de type Meltblown). Le non-tissé de type Spunmelt est un non-tissé formé à partir de filaments obtenus par extrusion.

L'activation de la nappe de non-tissé de type Spunmelt permet d'allonger et/ou casser les filaments de la nappe de non-tissé. On augmente ainsi la capacité d'élongation de la nappe de non-tissé.

Le présent exposé concerne également un procédé de fabrication d'un ensemble laminé s'étendant selon une direction longitudinale et une direction latérale, orthogonale à la direction longitudinale, le procédé étant défini par les revendications et comprenant les étapes suivantes :

- activation entre 20 et 200 %, dans la direction latérale, d'une zone d'une nappe de non-tissé s'étendant sur une longueur de la nappe de non-tissé mesurée dans la direction longitudinale et sur une largeur strictement inférieure à une largeur de la nappe de non-tissé mesurée dans la direction latérale ;
- fourniture d'un film élastique ;
- laminage de la nappe de non-tissé et du film élastique.

Le taux d'activation de la zone activée de la nappe de non-tissé dans la direction latérale peut être compris entre 40 et 200 %, en particulier entre 40 et 160 %.

Le laminage de la nappe de non-tissé et du film élastique permet d'assembler et de solidariser la nappe de non-tissé et le film élastique. L'assemblage et la solidarisation de la nappe de non-tissé et du film élastique peut se faire avec ou sans colle.

Par exemple, on peut envisager d'encapsuler les fibres et/ou les filaments de la nappe de non-tissé dans le film élastique lorsque la nappe de non-tissé est laminée avec le film élastique alors que ce dernier n'est pas encore complètement refroidi après extrusion.

On peut également envisager d'assembler la nappe de non-tissé et le film élastique par laminage sur toute la largeur de la nappe de non-tissé ou du film élastique ou par laminage sur une partie seulement de largeur de la nappe de non-tissé ou du film élastique, par exemple par soudure ultrasons, par calandrage à chaud ou à froid.

Le procédé peut comprendre, préalablement au laminage de la nappe de non-tissé, une étape d'enduction avec une colle de la nappe de non-tissé.

La colle peut être déposée sur la nappe de non-tissé de manière continue dans la direction longitudinale. Elle peut également être déposée sur la nappe de non-tissé de manière discontinue dans la direction longitudinale.

Dans la zone activée de la nappe de non-tissé, la colle peut être déposée de manière discontinue dans la direction latérale. Dans la zone activée de la nappe de non-tissé, la colle forme ainsi une pluralité de lignes de colle, par exemple continues dans la direction longitudinale, dont la largeur est inférieure à la largeur de la zone activée.

On peut adapter la largeur des lignes de colle et leur espacement dans la direction latérale.

Le procédé peut comprendre, préalablement au laminage de la nappe de non-tissé, une étape d'enduction avec une colle du film élastique.

La nappe de non-tissé est une première nappe de non-tissé, le procédé de fabrication comprend une étape de fourniture d'une deuxième nappe de non-tissé et une étape de laminage de la deuxième nappe de non-tissé sur le film élastique, ou de laminage de la deuxième nappe de non-tissé sur la première nappe de non-tissé. laminage de la deuxième nappe de non-tissé peut être réalisé lors du laminage avec l'élastique ou être réalisé lors d'une étape séparée, préalablement ou postérieurement au laminage avec l'élastique.

L'étape de laminage de la deuxième nappe de non-tissé sur le film élastique peut être réalisée simultanément à l'étape de laminage de la première nappe de non-tissé sur le film élastique. Ces étapes de laminage peuvent également être réalisées successivement.

Le film élastique peut être formé par une colle élastique.

La colle élastique peut être extrudée sur la première nappe de non-tissé et la première nappe de non-tissé enduite de la colle élastique peut être ensuite laminé avec la deuxième nappe de non-tissé ou inversement.

La colle élastique peut être également extrudée directement entre les deux nappes de non-tissé et ensuite, les deux nappes de non-tissé et la colle élastique sont laminés l'un avec l'autre.

Préalablement au laminage de la deuxième nappe de non-tissé, la deuxième nappe de non-tissé peut être activée entre 20 et 200 %, dans la direction latérale, sur une zone s'étendant sur une longueur de la nappe de non-tissé mesurée dans la direction longitudinale et sur une largeur strictement inférieure à une largeur de la nappe de non-tissé mesurée dans la direction latérale.

Le taux d'activation de la zone activée de la deuxième nappe de non-tissé dans la direction latérale peut être compris entre 40 et 200 %, en particulier entre 40 et 160 %.

Le procédé peut comprendre, préalablement au laminage de la deuxième nappe de non-tissé, une étape d'enduction avec une colle de la deuxième nappe de non-tissé.

La colle peut être déposée sur la nappe de non-tissé de manière continue dans la direction longitudinale. Elle peut également être déposée sur la nappe de non-tissé de manière discontinue dans la direction longitudinale.

Dans la zone activée de la nappe de non-tissé, la colle peut être déposée de manière discontinue dans la direction latérale. Dans la zone activée de la nappe de non-tissé, la colle forme ainsi une pluralité de lignes de colle, par exemple continues dans la direction longitudinale, dont la largeur est inférieure à la largeur de la zone activée.

L'ensemble laminé peut être activé à moins de 200 % dans la direction latérale, en particulier à moins de 150%, plus particulièrement à moins de 125%, encore plus particulièrement à moins de 100%.

Brève description des dessins

Description détaillée de l'invention

D'autres caractéristiques et avantages de l'invention ressortiront de la description suivante de modes de réalisation de l'invention, donnés à titre d'exemples non limitatifs, en référence aux figures annexées, sur lesquelles :

- la figure 1A est une vue schématique éclatée en coupe d'un ensemble laminé selon un premier mode de réalisation non couvert par l'invention ;
- la figure 1B est une variante du premier mode de réalisation non couvert par l'invention ;
- la figure 2 est une vue schématique éclatée en coupe d'un ensemble laminé selon un deuxième mode de réalisation non couvert par l'invention ;
- la figure 3A est une vue schématique éclatée en coupe d'un ensemble laminé selon un troisième mode de réalisation ;
- les figures 3B et 3C sont des variantes du troisième mode de réalisation ;
- la figure 4 est une vue schématique éclatée en coupe d'un ensemble laminé selon un quatrième mode de réalisation ;
- la figure 5 est une vue schématique éclatée en coupe d'un ensemble laminé selon un cinquième mode de réalisation ;
- la figure 6A est une vue schématique éclatée en coupe d'un ensemble laminé selon un sixième mode de réalisation ;
- la figure 6B est une variante du sixième mode de réalisation ;
- la figure 7 est vue schématique d'une installation de traitement d'une nappe de non-tissé, en vue de côté ;
- la figure 8 est une vue schématique, en vue du dessus, de la nappe de non-tissé défilant à un instant t dans l'installation de traitement de la figure 7 ;
- la figure 9 est une vue schématique du module d'activation de la figure 7 ;
- la figure 10 est un diagramme présentant les étapes d'un procédé de fabrication d'un ensemble laminé.

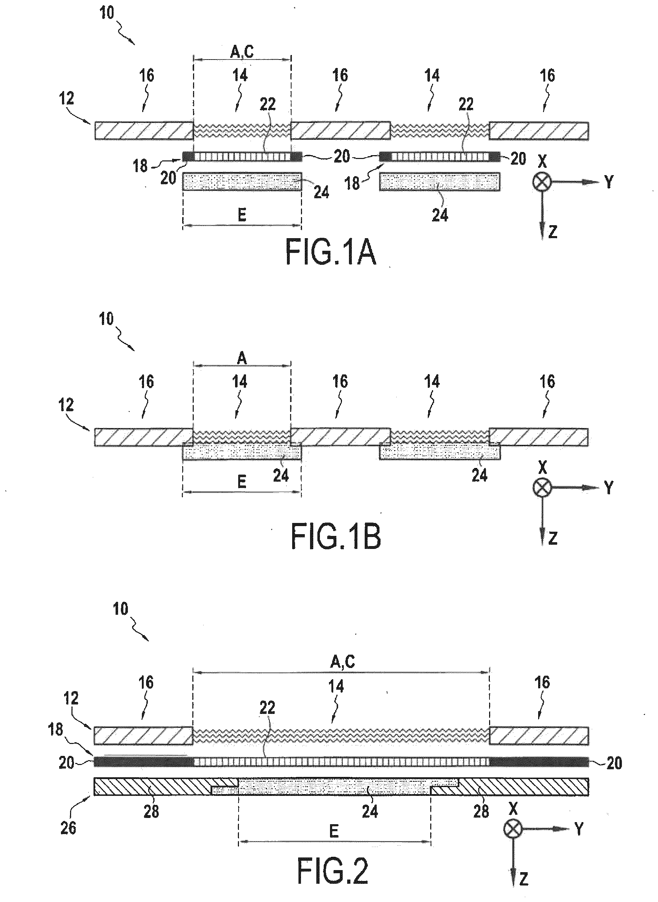

La figure 1A est une vue schématique en coupe selon le plan de coupe YZ d'un ensemble laminé 10 selon un premier mode de réalisation. La figure 1A est une vue éclatée de l'ensemble 10 afin de montrer chaque élément de l'ensemble laminé 10.

Cet ensemble laminé 10 comporte une nappe de non-tissé 12 qui s'étend selon une direction longitudinale X et une direction latérale Y, orthogonale à la direction longitudinale X. La nappe de non-tissé 12 comporte, sur la largeur de la nappe de non-tissé 12, deux zones activées 14 séparées par une zone non activée 16. La nappe de non-tissé 12 comporte également sur chaque bord latéral de la nappe de non-tissé 12 une zone non activée 16. On entend par zone activées 14 une zone activée 14 préalablement au laminage. On parlera également de zone activée préalablement 14.

La nappe de non-tissé 12 peut par exemple être un non-tissé cardé calandré, un non-tissé de type Spunlace, un non-tissé de type Spunmelt.

On comprend que les zones activées 14 ont une largeur strictement inférieure à la largeur de la nappe de non-tissé, la largeur étant mesurée dans la direction latérale Y. Les zones activées 14 s'étendent sur la longueur de la nappe de non-tissé 12, la longueur étant mesurée dans la direction longitudinale X.

L'ensemble laminé 10 comporte également deux films élastiques 24 qui sont reliés sur la nappe de non-tissé 12 par de la colle 18. La colle 18 est appliquée en bandes pleines 20 sur les bordures latérales des films élastiques 24 et en lignes étroites ou filets 22 sur les zones activées 14 de la nappe de non-tissé 12.

Comme matériau pour le film élastique 24, on peut par exemple envisager d'utiliser des matériaux élastiques comprenant une polyoléfine de type métallocène, ou à base de SIS ou de SBS. Le film élastique 24 peut être formé de trois couches ou plus ou être un skinlayer, c'est-à-dire un film élastique recouvert d'une peau.

On notera que dans le premier mode de réalisation de l'ensemble laminé 10, la largeur A de chaque zone activée 14 est inférieure à la largeur E de chaque film élastique 24.

La différence entre la largeur A et la largeur E peut être comprise entre 8 à 20 mm, par exemple cette différence peut être de 14 mm. Une telle différence permet d'obtenir une densité de fibres/filaments plus importante dans la zone non activée et autoriser une meilleure fixation entre le film élastique 24 et la nappe de non-tissé 12 correspondante. La somme des largeurs des zones activées peut être comprise entre 20 et 80 % de la largeur de la nappe de non-tissé 12 correspondante, en particulier entre 35 et 70 % de la largeur de la nappe de non-tissé 12.

Par ailleurs, la colle 18 est appliquée sur la nappe de non-tissé 12 sur une largeur égale à la largeur E du film élastique 24. La zone où la colle est appliquée en filets présente une largeur C inférieure à la largeur E du film élastique et égale sensiblement à la largeur A de la zone activée 14. Ainsi, la colle 18 étant disposée en bandes pleines 20 sur les bordures latérales des films élastiques 24 et des zones non activées 16 de la nappe de non-tissé 12, on assure une fixation ferme des films élastiques 24 sur la nappe de non-tissé.

Toutefois, on peut envisager que la largeur C de la zone où la colle est appliquée en filets soit différente de la largeur A de la zone activée.

Dans le premier mode de réalisation, la nappe de non-tissé 12 comporte deux zones activées 14, elle pourrait en comporter une seule. Elle pourrait également en comporter plus de deux.

Dans le premier mode de réalisation, les deux zones activées 14 ont la même largeur A. On pourrait bien entendu envisager que les deux zones activées 14 aient une largeur différente.

Par ailleurs, les films élastiques 24 ne sont pas activés. Le taux d'activation des zones activées 14 de la nappe de non-tissé 12 est donc supérieur au taux d'activation des films élastiques, ils sont donc différents.

On peut également envisager d'activer l'ensemble laminé 10 dans les zones de l'ensemble laminé où le film élastique 24 est présent. La nappe de non-tissé 12 ayant été activée avant le laminage de la nappe de non-tissé 12 avec le film élastique 24, c'est-à-dire préalablement au laminage, le taux d'activation des zones activées 14 de la nappe de non-tissé 12 sera modifié par l'activation de l'ensemble laminé 10. Toutefois, le taux d'activation résultant des zones activées 14 de la nappe de non-tissé dans la direction latérale Y restera toujours différent du taux d'activation du film élastique 24 dans la direction latérale Y, en particulier le taux d'activation résultant des zones activées 14 de la nappe de non-tissé dans la direction latérale Y sera supérieur du taux d'activation du film élastique 24 dans la direction latérale Y.

Cependant, grâce au fait que la nappe de non-tissé 12 a été activée préalablement au laminage avec les films élastiques 24, le taux d'activation appliqué à l'ensemble laminé 10 peut être inférieur au taux d'activation qu'il aurait fallu appliquer à l'ensemble laminé 10 si la nappe de non-tissé 12 n'avait pas été activée préalablement au laminage avec les films élastiques 24 pour obtenir une même capacité d'élongation de l'ensemble laminé 10. On réduit ainsi les risques de pollution de l'ensemble laminé 10 et/ou les risques de créer des trous, des coupures et/ou des fentes dans les films élastiques 24.

Par ailleurs, on peut également envisager que le taux d'activation de chaque zone activée 14 de la nappe de non-tissé 12 dans la direction latérale Y soit différent d'une zone activée à l'autre.

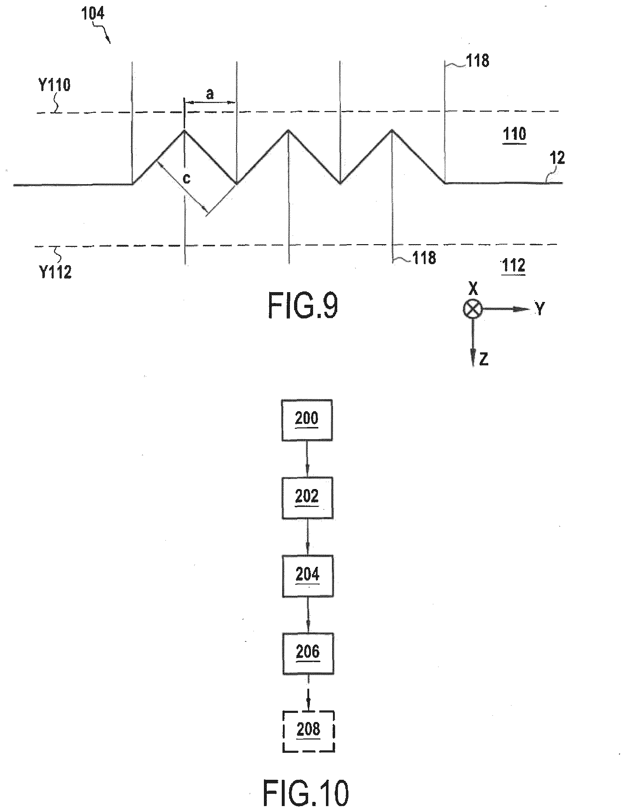

En référence à la figure 10, la nappe de non-tissé 12 est activée à l'étape 200 au cours de laquelle les deux zones activées 14 de la nappe de non-tissé 12 sont réalisées.

Ensuite, la colle 18 est déposée par enduction sur la nappe de non-tissé 12 à l'étape 202 et les films élastiques 24 sont rapportés sur la nappe de non-tissé 12 enduite de colle 18 à l'étape 204. La nappe de non-tissé 12 enduite de colle 18 et les films élastiques 24 sont laminés l'un avec l'autre à l'étape 206 afin de former un ensemble laminé 10. On pourrait en variante envisager de déposer la colle 18 sur le film élastique 24 au lieu de déposer de la colle 18 sur la nappe de non-tissé 12.

L'ensemble laminé 10 peut ensuite subir une activation de l'ensemble laminé 10 dans la direction latérale Y à l'étape 208.

Dans la variante du premier mode de réalisation représentée à la figure 1B, l'ensemble laminé 10 ne comporte pas de colle 18. La figure 1B n'est pas une vue explosée de l'ensemble laminé 10.

Dans cette variante, l'étape 202 n'est pas réalisée. En effet, dans cette variante, le film élastique 24 est extrudé et, alors que le film élastique 24 n'est pas complètement refroidi, on lamine la nappe de non-tissé 12 avec le film élastique 24 de sorte que les fibres et/ou les filaments de la nappe de non-tissé 12 soient encapsulées dans le film élastique 24.

Dans ce qui suit, les éléments communs aux différents modes de réalisation sont identifiés par les mêmes références numériques et ne seront pas décrits plus en détail.

Dans le deuxième mode de réalisation représenté à la figure 2, la nappe de non-tissé 12 de l'ensemble laminé 10 comporte une zone activée 14 disposée entre deux zones non activées 16. Les zones non activées 16 sont présentes sur chaque bord latéral de la nappe de non-tissé 12.

L'ensemble laminé comporte un film 26 comportant le film élastique 24 qui a été co-extrudé avec un matériau plastique non élastique 28, par exemple un matériau à base de polyéthylène.

Dans le deuxième mode de réalisation, la largeur A de la zone activée 14 est égale à la largeur C de la zone où la colle est appliquée en filets 22 et la largeur E du film élastique 24 est inférieure à la largeur A de la zone activée 14. Cependant, la largeur C pourrait être inférieure à la largeur E. Ainsi, la colle 18 étant disposée en bandes pleines 20 sur les bordures latérales du film 26, on assure une fixation ferme du film 26 sur la nappe de non-tissé 12.

Par ailleurs, le film 26 pourrait comporter plusieurs films élastiques 24, chaque film élastique étant séparé de l'autre par un matériau plastique non élastique 28 co-extrudé avec les films élastiques 24. Avec une pluralité de films élastiques 24, la nappe de non-tissé 12 pourrait alors comporter plusieurs zones activées 14 disposées en vis-à-vis des films élastiques 24 ou la nappe de non-tissé 12 pourrait ne présenter qu'une zone activée 14.

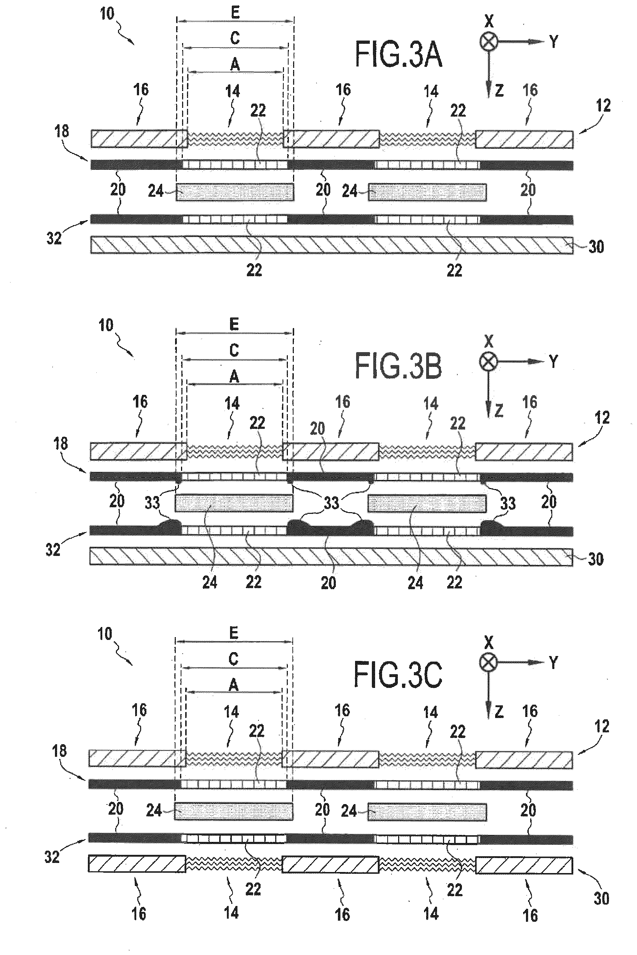

Dans le troisième mode de réalisation représenté à la figure 3A, l'ensemble laminé 10 comporte deux nappes de non-tissé 12, 30. La première nappe de non-tissé 12 est similaire à la nappe de non-tissé 12 de la figure 1A. La deuxième nappe de non-tissé 30 ne présente pas de zone activée préalablement au laminage. La deuxième nappe de non-tissé 30 peut être un non-tissé de type Spunlace.

Les films élastiques 24 sont reliés sur la deuxième nappe de non-tissé 30 par de la colle 32. Tout comme la colle 18, la colle 32 est appliquée en bandes pleines 20 sur les bordures latérales des films élastiques 24 et en lignes étroites ou filets 22 sur les zones activées 14 de la deuxième nappe de non-tissé 30.

Dans le troisième mode de réalisation (figures 3A, 3B et 3C), on notera que la largeur E du film élastique 24 est supérieure à la largeur A de la zone activée 14 et que la zone où la colle est appliquée en filets présente une largeur C inférieure à la largeur E du film élastique mais supérieure à la largeur A de la zone activée 14.

Tout comme dans le premier mode de réalisation, la première nappe de non-tissé 12 du troisième mode de réalisation comporte deux zones activées 14, elle pourrait en comporter une seule. Elle pourrait également en comporter plus de deux.

Dans le troisième mode de réalisation, les deux zones activées 14 ont la même largeur A. On pourrait bien entendu envisager que les deux zones activées 14 aient une largeur différente.

Les films élastiques 24 ne sont pas activés. Le taux d'activation des zones activées 14 de la première nappe de non-tissé 12 est donc supérieur au taux d'activation des films élastiques, ils sont donc différents. La deuxième nappe de non-tissé 30 n'est pas activée, si bien que le taux d'activation de la deuxième nappe de non-tissé, égal à zéro, est différent du taux d'activation des zones activées 14 de la première nappe de non-tissé 12.

On peut également envisager d'activer l'ensemble laminé 10 dans les zones de l'ensemble laminé où le film élastique 24 est présent. La première nappe de non-tissé 12 ayant été activée avant le laminage de la première nappe de non-tissé 12 avec le film élastique 24, c'est-à-dire préalablement au laminage, le taux d'activation des zones activées 14 de la première nappe de non-tissé 12 sera modifié par l'activation de l'ensemble laminé 10. Toutefois, le taux d'activation résultant des zones activées 14 de la première nappe de non-tissé 12 dans la direction latérale Y restera toujours différent du taux d'activation du film élastique 24 dans la direction latérale Y. Le taux d'activation de la deuxième nappe de non-tissé 30 est différent du taux d'activation des zones activées 14 de la première nappe de non-tissé 12, en particulier le taux d'activation résultant des zones activées 14 de la nappe de non-tissé 12 dans la direction latérale Y sera supérieur du taux d'activation du film élastique 24 dans la direction latérale Y.

En référence à la figure 10, on comprend qu'à l'étape 202, chaque nappe de non-tissé 12, 30 est enduite de colle 18 et qu'à l'étape 204, les films élastiques 24 sont rapportés entre les deux nappes de non-tissé 12, 30 avant que les deux nappes de non-tissé 12, 30 soient laminées avec les films élastiques 24 afin de former l'ensemble laminé 10 de la figure 3A.

Il est toutefois envisageable également qu'à l'étape 206, les films élastiques 24 soient laminés avec la première nappe de non-tissé 12 avant d'être ensuite laminés avec la deuxième nappe de non-tissé 30 ou inversement.

Dans la variante du troisième mode de réalisation représentée à la figure 3B, l'ensemble laminé 10 diffère de l'ensemble laminé de la figure 3A en ce que la colle 18, 32 présente des surépaisseurs 33 qui assurent une fixation encore plus ferme des films élastiques 24 sur les nappes de non-tissé 12, 30.

Dans la variante du troisième mode de réalisation représentée à la figure 3C, l'ensemble laminé 10 diffère de l'ensemble laminé de la figure 3A en ce que la deuxième nappe de non-tissé 30 est similaire à la nappe de non-tissé 12 de la figure 1A.

La deuxième nappe de non-tissé 30 pourrait comprendre plus de deux zones activées 14. Les zones activées 14 de la première nappe de non-tissé 12 pourraient présenter une largeur différente des zones activées 14 de la deuxième nappe de non-tissé 30. Par ailleurs, les zones activées 14 de chaque nappe de non-tissé 12, 30 pourraient présenter des largeurs différentes.

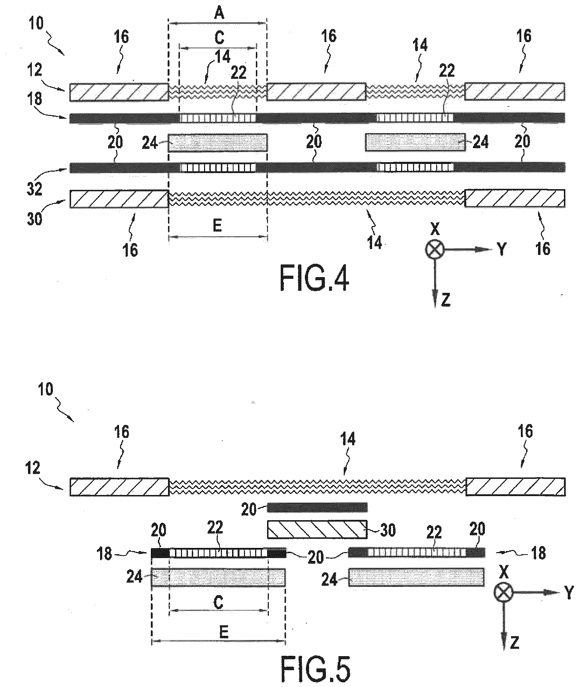

Dans le quatrième mode de réalisation représenté à la figure 4, l'ensemble laminé 10 comporte deux nappes de non-tissé 12, 30. La première nappe de non-tissé 12 est similaire à la nappe de non-tissé 12 de la figure 1A et la deuxième nappe de non-tissé 30 est similaire à la nappe de non-tissé 12 de la figure 2 qui comporte une zone activée 14.

On notera que dans le quatrième mode de réalisation de l'ensemble laminé 10, la largeur A de chaque zone activée 14 de la première nappe de non-tissé 12 est égale à la largeur E de chaque film élastique 24.

Par ailleurs, la colle 18 est appliquée sur toute la largeur de la nappe de non-tissé 12. La zone où la colle est appliquée en filets présente une largeur C inférieure à la largeur E du film élastique. Ainsi, la colle 18 étant disposée en bandes pleines 20 sur les bordures latérales des films élastiques 24, on assure une fixation ferme des films élastiques 24 sur la nappe de non-tissé.

Toutefois, on peut envisager que la largeur A des zones activées soit différente de la largeur des films élastiques E. La largeur A pourrait être inférieure ou supérieure à la largeur E.

Dans le quatrième mode de réalisation, la première nappe de non-tissé 12 comporte deux zones activées 14, elle pourrait en comporter une seule. Elle pourrait également en comporter plus de deux.

Dans le quatrième mode de réalisation, les deux zones activées 14 de la première nappe de non-tissé 12 ont la même largeur A. On pourrait bien entendu envisager que les deux zones activées 14 préalablement aient une largeur différente.

Dans le quatrième mode de réalisation, la deuxième nappe de non-tissé 30 comporte une zone activée 14, elle pourrait en comporter plus d'une.

Par ailleurs, les films élastiques 24 ne sont pas activés. Le taux d'activation des zones activées 14 de chaque nappe de non-tissé 12, 30 est donc supérieur au taux d'activation des films élastiques, les taux d'activation de chaque nappe de non-tissé 12, 30 dans la direction latérale Y sont donc différents du taux d'activation des films élastiques 24 dans la direction latérale Y. En outre, le taux d'activation de la première nappe de non-tissé 12 dans la direction latérale Y peut être différent du taux d'activation de la deuxième nappe de non-tissé 30 dans la direction latérale Y.

On peut également envisager d'activer l'ensemble laminé 10 dans les zones de l'ensemble laminé où le film élastique 24 est présent. Les nappes de non-tissé 12, 30 ayant été activées avant le laminage des nappes de non-tissé 12, 30 avec les films élastiques 24, c'est-à-dire préalablement au laminage, le taux d'activation des zones activées 14 de chaque nappe de non-tissé 12, 30 sera modifié par l'activation de l'ensemble laminé 10. Toutefois, le taux d'activation résultant des zones activées 14 de chaque nappe de non-tissé 12, 30 dans la direction latérale Y restera toujours différent du taux d'activation du film élastique 24 dans la direction latérale Y, en particulier le taux d'activation résultant des zones activées 14 de chaque nappe de non-tissé 12, 30 dans la direction latérale Y sera supérieur du taux d'activation du film élastique 24 dans la direction latérale Y.

Dans le cinquième mode de réalisation représenté à la figure 5, l'ensemble laminé 10 comporte deux nappes de non-tissé 12, 30. La première nappe de non-tissé 12 est similaire à la nappe de non-tissé 12 de la figure 2 et la deuxième nappe de non-tissé 30 est similaire à la deuxième nappe de non-tissé 30 de la figure 3A qui ne comporte pas de zone activée.

Dans le cinquième mode de réalisation, la deuxième nappe de non-tissé 30 est partiellement interposée entre la première nappe de non-tissé 12 et les films élastiques 24. La deuxième nappe de non-tissé 30 est dépourvue de zone activée et est enduite d'une bande pleine 20 de colle. La deuxième nappe de non-tissé 30 joue, dans ce cinquième mode de réalisation, un rôle de renfort de la première nappe de non-tissé 12, en particulier de la zone activée 14 de la première nappe de non-tissé 12.

Par exemple, la deuxième nappe de non-tissé 30 peut être un non-tissé cardé calandré ou un non-tissé de type Spunmelt. Comme représenté sur la figure 5, la deuxième nappe de non-tissé 30 présente ici une largeur bien inférieure à la largeur de la première nappe de non-tissé 12.

On notera que la colle 18 est appliquée en bandes pleines 20 entre les films élastiques 24 et les nappes de non-tissé 12, 30 de sorte que les bandes pleines 20 soient présentes sur les zones non activées des nappes de non-tissé 12,30. On assure ainsi une fixation ferme des films élastiques 24 sur les nappes de non-tissé 12, 30.

On notera, qu'entre la deuxième nappe de non-tissé 30 et les deux films élastiques 24, il n'y a pas de colle 18 disposée entre les deux films élastiques dans la direction latérale Y.

Tout comme dans les modes de réalisation précédents, on peut envisager de réaliser des largeurs A, E et D différentes, on peut également envisager un nombre différent de films élastiques 24 et/ou de zones activées 14.

On peut également envisager d'activer l'ensemble laminé 10 dans les zones de l'ensemble laminé où le film élastique 24 est présent. La première nappe de non-tissé 12 ayant été activée avant le laminage des nappes de non-tissé 12, 30 avec les films élastiques 24, c'est-à-dire préalablement au laminage, le taux d'activation de la zone activée 14 de la première nappe de non-tissé 12 sera modifié par l'activation de l'ensemble laminé 10. Toutefois, le taux d'activation résultant de la zone activée 14 de la première nappe de non-tissé 12 dans la direction latérale Y restera toujours différent du taux d'activation du film élastique 24 dans la direction latérale Y. La deuxième nappe de non-tissé 30 n'est pas activée de sorte que le taux d'activation de la deuxième nappe de non-tissé 30 dans la direction latérale Y, qui est égal à zéro, est différent du taux d'activation de la zone activée 14 de la première nappe de non-tissé 12 et est différent du taux d'activation du film élastique 24 dans la direction latérale Y, en particulier le taux d'activation résultant des zones activées 14 de la nappe de non-tissé 12 dans la direction latérale Y sera supérieur du taux d'activation du film élastique 24 dans la direction latérale Y.

En référence à la figure 10, on comprend qu'à l'étape 202, la première nappe de non-tissé 12 ou la deuxième nappe de non-tissé 30 est enduite d'une bande pleine 20 de colle 18 et que les films élastiques 24 sont également enduits de colle 18, qu'à l'étape 204, la deuxième nappe de non-tissé 30 est rapportée sur la première nappe de non-tissé 12 et que les films élastiques 24 sont rapportés sur les deux nappes de non-tissé 12, 30 de sorte que la deuxième nappe de non-tissé 30 est partiellement interposée entre la première nappe de non-tissé 12 et les films élastiques 24. Les deux nappes de non-tissé 12, 30 sont laminées avec les films élastiques 24 afin de former l'ensemble laminé 10 de la figure 5.

On comprend que la deuxième nappe de non-tissé 30 est laminée sur la première nappe de non-tissé 12. Cette étape peut être réalisée en même temps que le laminage des films élastiques 24 sur les deux nappes de non-tissé 12, 30. Ces deux laminages peuvent également être réalisés de manière consécutive. On pourrait également rapporter une troisième nappe de non-tissé sur laquelle de la colle pourrait être déposée et rapporter cette troisième nappe de non-tissé du côté des films élastiques 24 opposé aux premières et deuxièmes nappes de non-tissé 12, 30.

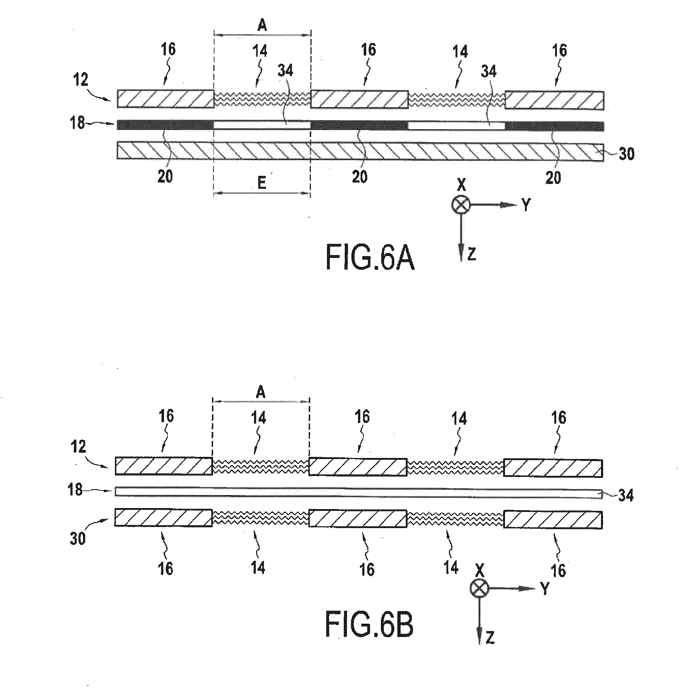

Dans le sixième mode de réalisation présenté à la figure 6A, l'ensemble laminé 10 comporte une première nappe de non-tissé 12 similaire à la nappe de non-tissé de la figure 1A et une deuxième nappe de non-tissé similaire à la deuxième nappe de non-tissé de la figure 3A.

Dans le sixième mode de réalisation, la colle 18 est directement enduite sur la première nappe de non-tissé 12. Cette colle comporte des bandes pleines 20 de colle et des bandes pleines 34 de colle élastique. Les bandes pleines 34 de colle élastique forment les films élastiques. La deuxième nappe de non-tissé 30 est ensuite rapportée sur la première nappe de non-tissé enduite de la colle comprenant des bandes pleines 34 de colle élastique, la colle 18 et donc les bandes pleines 34 de colle élastique sont interposées entre les nappes de non-tissé 12, 30.

Dans le sixième mode de réalisation, la largeur A de la zone activée 14 est égale à la largeur E des bandes pleines 34 de la colle élastique. On pourrait envisager que la largeur A soit supérieure ou inférieure à la largeur E.

Tout comme dans les modes de réalisation précédents, on peut également avoir un nombre différent de films élastiques 24 et/ou de zones activées 14. La deuxième nappe de non-tissé 30 peut comprendre une ou plusieurs zones activées 14.

On peut également envisager d'activer l'ensemble laminé 10 dans les zones de l'ensemble laminé où les bandes pleines 34 de colle élastique sont présentes. La première nappe de non-tissé 12 ayant été activée avant le laminage de la première nappe de non-tissé 12 avec la colle 18, le taux d'activation des zones activées 14 de la première nappe de non-tissé 12 sera modifié par l'activation de l'ensemble laminé 10. Toutefois, le taux d'activation résultant des zones activées 14 de la première nappe de non-tissé 12 dans la direction latérale Y restera toujours différent du taux d'activation du film élastique 24 dans la direction latérale Y.

Dans la variante du sixième mode de réalisation représentée à la figure 6B, l'ensemble laminé 10 diffère de l'ensemble laminé de la figure 6A en ce que la deuxième nappe de non-tissé 30 est similaire à la nappe de non-tissé 12 de la figure 1A et en ce que la colle 18 est constituée d'une seule bande pleine 34 de colle élastique. La bande pleine 34 de colle élastique forme le film élastique.

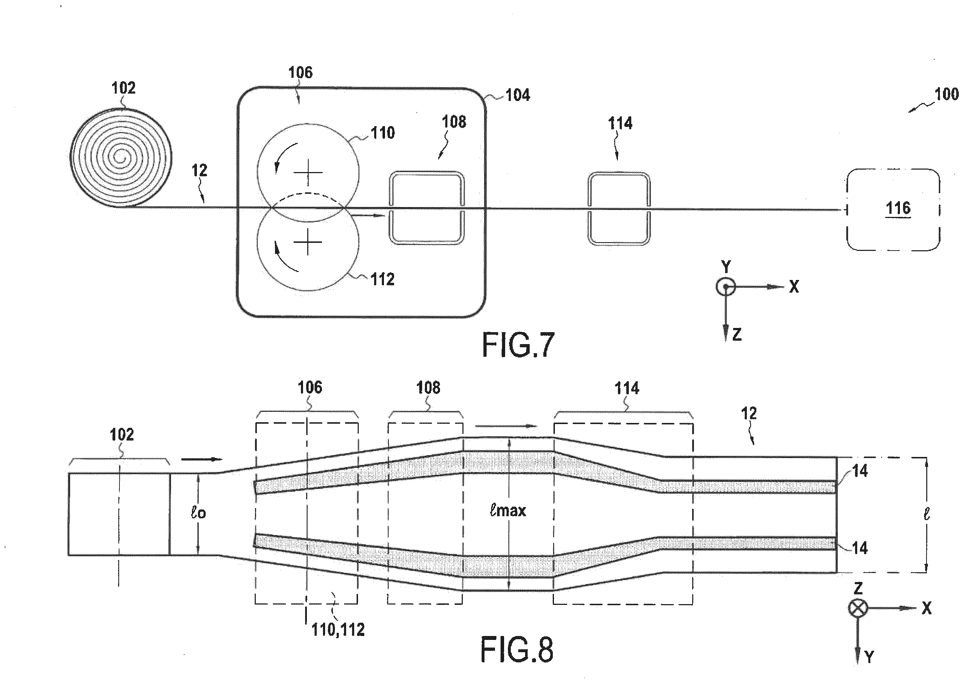

La figure 7 représente une installation de traitement 100 d'une nappe de non-tissé 12, pouvant notamment être utilisée en amont d'une ligne de production d'un ensemble laminé 10 pour la réalisation d'une nappe de non-tissé 12 et la figure 8 est une vue schématique, en vue du dessus, de la nappe de non-tissé 12 défilant à un instant t dans l'installation de traitement 100 de la figure 7 et sur laquelle les dimensions de la nappe de non-tissé 12 sont représentées schématiquement.

L'installation 100 comprend, de l'amont vers l'aval dans la direction de défilement de la nappe de non-tissé 12 à traiter (de la gauche vers la droite, sur la figure 7) :

- un poste de déroulement 102 de la nappe de non-tissé 12, initialement conditionnée sous forme de bobine,
- un module d'élargissement 104 de la nappe de non-tissé, comprenant :

  ○ un module d'activation 106 destiné à activer des zones localisées de la nappe de non-tissé 12, formant les zones activées 14 de la nappe de non-tissé 12, et
  ○ un module d'étirement 108 pour étirer la nappe de non-tissé 12 dans sa direction latérale Y, et

- un module de gestion de la largeur 114 de la nappe de la nappe de non-tissé 12.

La nappe de non-tissé 12, une fois déroulée, est activée localement par le module d'activation 106 (étape 200).

La figure 9 représente une vue schématique en coupe selon le plan YZ du module d'activation 106. Le module d'activation 106 comprend deux rouleaux d'activation 110, 112 munis chacun d'un empilement de disques 118 parallèles et espacés les uns des autres d'une distance 2a. Les rouleaux d'activation 110, 112 et les disques 118 comprennent chacun un axe de symétrie Y110, Y112 qui est orienté selon la direction latérale Y. Les disques 118 du premier rouleau d'activation 110 sont disposés dans les espaces inter-disques du deuxième rouleau d'activation 112 et les axes de symétrie Y110, Y112 des deux rouleaux d'activation 110, 112 sont espacés l'un de l'autre d'une distance inférieure au diamètre des disques 118 de sorte que les zones de la nappe de non-tissé 12 passant entre les disques 12 des rouleaux d'activation 110, 112 sont étirées dans la direction latérale Y, formant les zones activées 14. A ces endroits, les fibres et/ou filaments de la nappe de non-tissé 12 sont étirés et/ou cassés. On comprend que plus la distance entre les axes de symétrie Y110, Y112 des deux rouleaux d'activation 110, 112 est faible, plus l'interpénétration des disques 118 du premier rouleau d'activation 110 par rapport au deuxième rouleau d'activation 112 est grande et plus le taux d'activation est important.

De manière connue, le taux d'activation est le rapport de la différence entre la distance c entre l'extrémité de deux disques adjacent appartenant chacun à un rouleau d'activation différent et la distance a entre l'extrémité des deux mêmes disques projetée sur l'axe de symétrie d'un rouleau d'activation rapportée à cette distance a entre l'extrémité des deux mêmes disques projetée sur l'axe de symétrie d'un rouleau d'activation exprimé en pourcent.

Le taux d'activation, en pourcentage, peut être exprimé comme suit :

$$\text{Taux d'activation} = [(c-a)/a]*100$$

La nappe de non-tissé 12 est ensuite déformée plus globalement par le module d'étirement 108 qui comprend généralement une pluralité de rouleaux (non représentés) ayant pour but d'étirer latéralement la nappe de non-tissé. Elle y est étirée jusqu'à prendre une largeur lmax supérieure à la largeur utile l souhaitée pour l'ensemble laminé fabriqué en aval sur la ligne de production 116.

Dans le module de gestion de la largeur 114, qui comprend des moyens de mesure de la largeur de la nappe de non-tissé 12 et des moyens d'ajustement, par exemple par étirage, de cette largeur à une valeur souhaitée, la largeur de la nappe de non-tissé 12 est ajustée à la largeur utile l qu'elle devra conserver sur toute la ligne de production 116.

Exemples

Plusieurs ensembles laminés 10 ont été réalisés.

Exemples 1 et 2 : l'ensemble laminé 10 comporte deux nappes de non-tissé, une première nappe de non-tissé cardé calandré comprenant des zones activées préalablement 14 à un taux d'activation de 153 % et une deuxième nappe de non-tissé de type Spunlace comprenant des zones activées préalablement 14 à un taux d'activation de 44 %.

Exemple 3 : l'ensemble laminé 10 comporte deux nappes de non-tissé, une première nappe de non-tissé cardé calandré comprenant des zones activées préalablement 14 à un taux d'activation de 106 % et une deuxième nappe de non-tissé de type Spunlace comprenant des zones activées préalablement 14 à un taux d'activation de 44 %.

Exemple 4 : l'ensemble laminé 10 comporte deux nappes de non-tissé, une première nappe de non-tissé cardé calandré comprenant des zones activées préalablement 14 à un taux d'activation de 153 % et une deuxième nappe de non-tissé de type Spunlace dépourvue de zone activée préalablement.

Exemple 5 : l'ensemble laminé 10 comporte deux nappes de non-tissé, une première nappe de non-tissé cardé calandré comprenant des zones activées préalablement 14 à un taux d'activation de 106% et une deuxième nappe de non-tissé de type Spunlace dépourvue de zone activée préalablement.

Exemple 6 : l'ensemble laminé 10 comporte deux nappes de non-tissé, une première nappe de non-tissé cardé calandré comprenant des zones activées préalablement 14 à un taux d'activation de 153 % et une deuxième nappe de non-tissé de type Spunlace dépourvue de zone activée préalablement et l'ensemble laminé 10 est ensuite activé à un taux d'activation de 88 %.

On a ensuite réalisé deux exemples de contrôle dans lesquels aucune nappe de non-tissé n'est activée préalablement au laminage. Ces exemples 7 et 8 comportent chacun deux nappes de non-tissé, une première nappe de non-tissé cardé calandré dépourvue de zone activée et une deuxième nappe de non-tissé de type Spunlace dépourvue de zone activée et l'ensemble laminé est ensuite activé à un taux d'activation respectivement de 110 % et de 93 %.

Tous les exemples ont été fabriqués à des vitesses de défilement sur la ligne de production 116 supérieures à 200 m/min (mètre/minutes) à l'exception des exemples 2 et 3 qui ont été fabriqués à des vitesses de défilement sur la ligne de production 116 inférieures à 200 m/min.

On a ensuite mesuré l'allongement à 10 N (Newton) exprimé en millimètres (mm) des exemples 1-8, les échantillons de mesure ayant une largeur de 50 mm, l'étirement étant réalisé dans la direction latérale Y sur une largeur de 30 mm à une vitesse de 100 mm/min (millimètre/minute). La largeur de 30 mm est choisie de sorte que seule la zone activée est testée. On a également mesuré la valeur de la force maximum (en N) avant rupture de chacun des échantillons. Les résultats sont présentés dans le tableau 1 ci-dessous.

Tableau 1

| Exemple | Allongement à 10 N (en mm) | Force maximum (en N) |
|---|---|---|
| Exemple 1 | 25,0 | 38 |
| Exemple 2 | 25,1 | 38 |
| Exemple 3 | 20,2 | 40 |
| Exemple 4 | 21,2 | 41 |
| Exemple 5 | 19,7 | 42 |
| Exemple 6 | 27,7 | 37 |
| Exemple 7 | 28,0 | 35 |
| Exemple 8 | 24,5 | 37 |

On constate que grâce à l'activation d'au moins une nappe de non-tissé préalablement au laminage, on obtient des ensembles laminés 10 (exemples 1-6) présentant un allongement à 10 N similaires aux valeurs obtenues pour les exemples 7 et 8 sans activer l'ensemble laminé 10 pour les exemples 1 à 5. On constate également que la force maximum que peuvent subir les ensembles laminés 10 avant rupture (exemples 1-6) est supérieure à la force maximum que peuvent subir les ensembles laminés des exemples 7 et 8. Les ensembles laminés 10 des exemples 1-6 peuvent donc subir des forces de traction dans la direction latérale Y qui sont supérieures aux forces de traction dans la direction latérale Y que peuvent subir les ensembles laminés des exemples 7 et 8.

Par ailleurs, on note que la vitesse de défilement sur la ligne de production 116 des nappes de non-tissé n'influence pas les propriétés de l'ensemble laminé 10 (voir notamment les exemples 1 et 2). Ainsi, bien que l'on travaille avec des nappes de non-tissé préalablement activées, il n'est pas nécessaire de réduire la vitesse de défilement sur la ligne de production. De plus, on constate que l'ensemble laminé de l'exemple 4 présente une texture/un visuel plus uniforme que la texture/le visuel de l'ensemble laminé de l'exemple 8. L'ensemble laminé de l'exemple 8 présente une texture/un visuel plus uniforme que la texture/le visuel de l'ensemble laminé de l'exemple 6. On entend par texture/visuel plus uniforme, le fait que l'ensemble laminé comprend moins de zones avec un contraste différent lorsque l'ensemble laminé est étiré. Par exemple, pour une valeur d'étirage obtenue sous une force de 10 N dans le sens CD, conformément au sigle anglais pour « Cross Direction », c'est-à-dire dans la direction latérale Y, pour un échantillon ayant une dimension en CD de 35 mm et 50 mm et une dimension en MD, c'est-à-dire dans la direction longitudinale X, conformément au sigle anglais pour « Machine Direction », comprise entre 50 mm et 100 mm.

Des mesures d'allongement à 5 N ont également été réalisées sur les nappes de non-tissé seules avant le laminage. Ces mesures d'allongement à 5 N ont été réalisées avec une pré-charge de 0,1 N, à une vitesse de 500 mm/min. Les mesures d'allongement ont été réalisées dans la direction latérale Y, aussi identifiée CD ainsi que dans la direction longitudinale X, aussi identifiée MD.

Les résultats sont présentés dans le tableau 2 ci-dessous, dans lequel l'allongement à 5 N est exprimé en % de la longueur initiale de la nappe de non-tissé.

Le non-tissé 1 est un non-tissé cardé calandré activé préalablement à 153 % de 22 $g/m^2$ (gramme/mètre au carré).

Le non-tissé 2 est un non-tissé cardé calandré activé préalablement à 106 % de 22 $g/m^2$.

Le non-tissé 3 est un non-tissé cardé calandré non activé préalablement de 22 $g/m^2$.

Le non-tissé 4 est un non-tissé de type Spunlace activé préalablement à 44 % de 30 $g/m^2$.

Le non-tissé 5 est un non-tissé de type Spunlace non activé préalablement de 30 $g/m^2$.

Tableau 2

| Exemple | Sens | Allongement à 5 N (en %) |
|---|---|---|
| Non-tissé 1 | CD | 117 |
| Non-tissé 2 | CD | 72 |

(suite)

| Exemple | Sens | Allongement à 5 N (en %) |
|---|---|---|
| Non-tissé 3 | CD | 78 |
| Non-tissé 4 | CD | 105 |
| Non-tissé 5 | CD | 102 |
| Non-tissé 1 | MD | 5,4 |
| Non-tissé 2 | MD | 4,1 |
| Non-tissé 3 | MD | 2,8 |
| Non-tissé 4 | MD | 4,4 |
| Non-tissé 5 | MD | 3,0 |

Les non-tissés 1 à 5 présentent un grammage compris entre 10 g (gramme) et 40 g.

On constate que le non-tissé 1 présente une élongation similaire au non-tissé 4 ou au non-tissé 5, dans le sens CD.

Quoique le présent exposé ait été décrit en se référant à des exemples de réalisation spécifiques, il est évident que des différentes modifications et changements peuvent être effectués sur ces exemples sans sortir de la portée générale de l'invention telle que définie par les revendications. En outre, des caractéristiques individuelles des différents modes de réalisation évoqués peuvent être combinées dans des modes de réalisation additionnels. Par conséquent, la description et les dessins doivent être considérés dans un sens illustratif plutôt que restrictif. Ainsi, nous pourrions envisager un autre couple pour la première et la deuxième nappe de non-tissé 12, 30, par exemple :

- un non-tissé cardé calandré et un non-tissé Spunlace, ou
- un non-tissé Spunlace et un non-tissé Spunlace, ou
- un non-tissé cardé calandré et un non-tissé Spunmelt, ou
- un non-tissé Spunmelt et un non-tissé Spunlace.

Pour les exemples mentionnés précédemment, la prei-ère et la deuxième nappe de non-tissé peuvent être interverties.

## Revendications

1. Ensemble laminé (10) s'étendant selon une direction longitudinale (X) et une direction latérale (Y), orthogonale à la direction longitudinale (X), l'ensemble comprenant une première nappe de non-tissé (12) et un film élastique (24) laminés ensemble, la première nappe de non-tissé (12) comprenant au moins une zone activée (14) s'étendant sur une longueur de la nappe de non-tissé mesurée dans la direction longitudinale (X) et sur une largeur strictement inférieure à une largeur de la nappe de non-tissé mesurée dans la direction latérale (Y), le taux d'activation de la zone activée (14) de la première nappe de non-tissé (12) dans la direction latérale (Y) étant différent du taux d'activation du film élastique (24) dans la direction latérale (Y), le taux d'activation de la zone activée (14) de la première nappe de non-tissé (12) dans la direction latérale (Y) étant compris entre 20 et 200 %, l'ensemble laminé (10) comprenant une deuxième nappe de non-tissé (30),
le film élastique (24) étant interposé entre la première et la deuxième nappe de non-tissé (12, 30),
dans lequel le taux d'activation de la zone activée (14) de la première nappe de non-tissé (12) dans la direction latérale (Y) est différent du taux d'activation de la zone activée (14) de la deuxième nappe de non-tissé (30) dans la direction latérale (Y).

2. Ensemble laminé (10) s'étendant selon une direction longitudinale (X) et une direction latérale (Y), orthogonale à la direction longitudinale (X), l'ensemble comprenant une première nappe de non-tissé (12) et un film élastique (24) laminés ensemble, la première nappe de non-tissé (12) comprenant au moins une zone activée (14) s'étendant sur une longueur de la nappe de non-tissé mesurée dans la direction longitudinale (X) et sur une largeur strictement inférieure à une largeur de la nappe de non-tissé mesurée dans la direction latérale (Y), le taux d'activation de la zone activée (14) de la première nappe de non-tissé (12) dans la direction latérale (Y) étant différent du taux d'activation du film élastique (24) dans la direction latérale (Y), le taux d'activation de la zone activée (14) de la première nappe de non-tissé (12) dans la direction latérale (Y) étant compris entre 20 et 200 %l'ensemble laminé (10) comprenant une deuxième nappe de non-tissé (30) et la deuxième nappe de non-tissé (30) étant au moins partiellement interposée entre la première nappe de non-tissé (12) et le film élastique (24),

dans lequel le taux d'activation de la zone activée (14) de la première nappe de non-tissé (12) dans la direction latérale (Y) est différent du taux d'activation de la zone activée (14) de la deuxième nappe de non-tissé (30) dans la direction latérale (Y).

3. Ensemble laminé (10) selon l'une des revendications 1 ou 2, dans lequel la deuxième nappe de non-tissé (30) est dépourvue de zone activée.

4. Ensemble laminé (10) selon l'une des revendications 1 ou 2 , dans lequel la deuxième nappe de non-tissé (30) comprend au moins une zone activée (14) s'étendant sur une longueur de la nappe de non-tissé mesurée dans la direction longitudinale (X) et sur une largeur strictement inférieure à une largeur de la nappe de non-tissé mesurée dans la direction latérale (Y), le taux d'activation de la zone activée (14) de la deuxième nappe de non-tissé (30) dans la direction latérale (Y) étant compris entre 20 et 200 %.

5. Ensemble laminé (10) selon l'une quelconque des revendications 1 à 4, dans lequel le taux d'activation de la zone activée (14) de la deuxième nappe de non-tissé (30) dans la direction latérale (Y) est différent du taux d'activation du film élastique (24) dans la direction latérale (Y).

6. Ensemble, laminé (10) selon l'une quelconque des revendications 1 à 5, dans lequel la nappe de non-tissé (12, 30) est un non-tissé cardé calandré.

7. Ensemble laminé (10) selon l'une quelconque des revendications 1 à 5, dans lequel la nappe de non-tissé (12, 30) est un non-tissé de type Spunlace.

8. Ensemble laminé (10) selon l'une quelconque des revendications 1 à 5, dans lequel la nappe de non-tissé (12, 30) est un non-tissé de type Spunmelt.

9. Procédé de fabrication d'un ensemble laminé (10) s'étendant selon une direction longitudinale (X) et une direction latérale (Y), orthogonale à la direction longitudinale (X), le procédé comprenant les étapes suivantes :

   - activation entre 20 et 200 %, dans la direction latérale (Y), d'une zone d'une première nappe de non-tissé (12) s'étendant sur une longueur de la première nappe de non-tissé mesurée dans la direction longitudinale (X) et sur une largeur strictement inférieure à une largeur de la première nappe de non-tissé mesurée dans la direction latérale (Y) ;
   - fourniture d'un film élastique (24) ;
   - laminage de la première nappe de non-tissé (12) et du film élastique (24),
   - fourniture d'une deuxième nappe de non-tissé (30),
   - laminage de la deuxième nappe de non-tissé (30) sur le film élastique (24)

   dans lequel le taux d'activation de la zone activée (14) de la première nappe de non-tissé (12) dans la direction latérale (Y) est différent du taux d'activation de la zone activée (14) de la deuxième nappe de non-tissé (30) dans la direction latérale (Y).

10. Procédé de fabrication d'un ensemble laminé (10) s'étendant selon une direction longitudinale (X) et une direction latérale (Y), orthogonale à la direction longitudinale (X), le procédé comprenant les étapes suivantes :

   - activation entre 20 et 200 %, dans la direction latérale (Y), d'une zone d'une première nappe de non-tissé (12) s'étendant sur une longueur de la première nappe de non-tissé mesurée dans la direction longitudinale (X) et sur une largeur strictement inférieure à une largeur de la première nappe de non-tissé mesurée dans la direction latérale (Y) ;
   - fourniture d'un film élastique (24) ;
   - laminage de la première nappe de non-tissé (12) et du film élastique (24),
   - fourniture d'une deuxième nappe de non-tissé (30),
   - laminage de la deuxième nappe de non-tissé (30) sur la première nappe de non-tissé (12)

   dans lequel le taux d'activation de la zone activée (14) de la première nappe de non-tissé (12) dans la direction latérale (Y) est différent du taux d'activation de la zone activée (14) de la deuxième nappe de non-tissé (30) dans la direction latérale (Y).

**11.** Procédé de fabrication selon l'une des revendications 9 ou 10, comprenant, préalablement au laminage de la nappe de non-tissé (12), une étape d'enduction avec une colle (18) de la nappe de non-tissé (12).

**12.** Procédé de fabrication selon l'une des revendications 9 à 11, dans lequel, préalablement au laminage de la deuxième nappe de non-tissé (30), la deuxième nappe de non-tissé (30) est activée entre 20 et 200 %, dans la direction latérale (Y), sur une zone s'étendant sur une longueur de la nappe de non-tissé mesurée dans la direction longitudinale (X) et sur une largeur strictement inférieure à une largeur de la nappe de non-tissé mesurée dans la direction latérale (Y).

**13.** Procédé de fabrication selon l'une des revendications 9 à 12, comprenant, préalablement au laminage de la deuxième nappe de non-tissé (30), une étape d'enduction avec une colle (18) de la deuxième nappe de non-tissé (30).

**14.** Procédé de fabrication selon l'une quelconque des revendications 9 à 13, dans lequel l'ensemble laminé (10) est activé à moins de 200 % dans la direction latérale (Y).

**Patentansprüche**

**1.** Laminat (10), das sich entlang einer Längsrichtung (X) und einer seitlichen Richtung (Y) erstreckt, die orthogonal zu der Längsrichtung (X) ist, wobei das Laminat eine erste Vliesmatte (12) und einen elastischen Film (24) umfasst, die zusammenlaminiert sind, wobei die erste Vliesmatte (12) mindestens eine aktivierte Zone (14) umfasst, die sich über eine in der Längsrichtung (X) gemessene Länge der Vliesmatte und über eine Breite erstreckt, die strikt kleiner als eine in der seitlichen Richtung (Y) gemessene Breite der Vliesmatte ist, wobei die Aktivierungsrate der aktivierten Zone (14) der ersten Vliesmatte (12) in der seitlichen Richtung (Y) sich von der Aktivierungsrate des elastischen Films (24) in der seitlichen Richtung (Y) unterscheidet, wobei die Aktivierungsrate der aktivierten Zone (14) der ersten Vliesmatte (12) in der seitlichen Richtung (Y) zwischen 20 und 200 % beträgt, wobei das Laminat (10) eine zweite Vliesmatte (30) umfasst, der elastische Film (24) zwischen die erste und die zweite Vliesmatte (12, 30) zwischengelegt ist, wobei sich die Aktivierungsrate der aktivierten Zone (14) der ersten Vliesmatte (12) in der seitlichen Richtung (Y) von der Aktivierungsrate des aktivierten Zone (14) der zweiten Vliesmatte (30) in der seitlichen Richtung (Y) unterscheidet.

**2.** Laminat (10), das sich entlang einer Längsrichtung (X) und einer seitlichen Richtung (Y) erstreckt, die orthogonal zu der Längsrichtung (X) ist, wobei das Laminat eine erste Vliesmatte (12) und einen elastischen Film (24) umfasst, die zusammenlaminiert sind, wobei die erste Vliesmatte (12) mindestens eine aktivierte Zone (14) umfasst, die sich über eine in der Längsrichtung (X) gemessene Länge der Vliesmatte und über eine Breite erstreckt, die strikt kleiner als eine in der seitlichen Richtung (Y) gemessene Breite der Vliesmatte ist, wobei die Aktivierungsrate der aktivierten Zone (14) der ersten Vliesmatte (12) in der seitlichen Richtung (Y) sich von der Aktivierungsrate des elastischen Films (24) in der seitlichen Richtung (Y) unterscheidet, wobei die Aktivierungsrate der aktivierten Zone (14) der ersten Vliesmatte (12) in der seitlichen Richtung (Y) zwischen 20 und 200 % beträgt, das Laminat (10) umfassend eine zweite Vliesmatte (30), und wobei die zweite Vliesmatte (30) zumindest teilweise zwischen die erste Vliesmatte (12) und den elastischen Film (24) zwischengelegt ist, wobei sich die Aktivierungsrate der aktivierten Zone (14) der ersten Vliesmatte (12) in der seitlichen Richtung (Y) von der Aktivierungsrate des aktivierten Zone (14) der zweiten Vliesmatte (30) in der seitlichen Richtung (Y) unterscheidet.

**3.** Laminat (10) nach einem der Ansprüche 1 oder 2, wobei die zweite Vliesmatte (30) keine aktivierte Zone aufweist.

**4.** Laminat (10) nach einem der Ansprüche 1 oder 2, wobei die zweite Vliesmatte (30) mindestens eine aktivierte Zone (14) umfasst, die sich über eine in der Längsrichtung (X) gemessenen Länge der Vliesmatte und über eine Breite erstreckt, die strikt kleiner als eine in der seitlichen Richtung (Y) gemessene Breite der Vliesmatte ist, wobei die Aktivierungsrate der aktivierten Zone (14) der zweiten Vliesmatte (30) in der seitlichen Richtung (Y) zwischen 20 und 200 % beträgt.

**5.** Laminat (10) nach einem der Ansprüche 1 bis 4, wobei sich die Aktivierungsrate der aktivierten Zone (14) der zweiten Vliesmatte (30) in der seitlichen Richtung (Y) von der Aktivierungsrate des elastischen Films (24) in der seitlichen Richtung (Y) unterscheidet.

6. Laminat (10) nach einem der Ansprüche 1 bis 5, wobei die Vliesmatte (12, 30) ein kalandriertes kardiertes Vlies ist.

7. Laminat (10) nach einem der Ansprüche 1 bis 5, wobei die Vliesmatte (12, 30) ein Vlies vom Typ Spunlace ist.

8. Laminat (10) nach einem der Ansprüche 1 bis 5, wobei die Vliesmatte (12, 30) ein Vlies vom Typ Spunmelt ist.

9. Verfahren zur Herstellung eines Laminats (10), das sich entlang einer Längsrichtung (X) und einer seitlichen Richtung (Y) erstreckt, die orthogonal zu der Längsrichtung (X) ist, wobei das Verfahren die folgenden Schritte umfasst:

   - Aktivieren von zwischen 20 und 200 % in der seitlichen Richtung (Y) einer Zone einer ersten Vliesmatte (12), die sich über eine in der Längsrichtung (X) gemessene Länge der ersten Vliesmatte und über eine Breite erstreckt, die strikt kleiner als eine in der seitlichen Richtung (Y) gemessene Breite der ersten Vliesmatte ist;
   - Bereitstellen eines elastischen Films (24);
   - Laminieren der ersten Vliesmatte (12) und des elastischen Films (24),
   - Bereitstellen einer zweiten Vliesmatte (30),
   - Laminieren der zweiten Vliesmatte (30) und des elastischen Films (24),

   wobei sich die Aktivierungsrate der aktivierten Zone (14) der ersten Vliesmatte (12) in der seitlichen Richtung (Y) von der Aktivierungsrate des aktivierten Zone (14) der zweiten Vliesmatte (30) in der seitlichen Richtung (Y) unterscheidet.

10. Verfahren zur Herstellung eines Laminats (10), das sich entlang einer Längsrichtung (X) und einer seitlichen Richtung (Y) erstreckt, die orthogonal zu der Längsrichtung (X) ist, wobei das Verfahren die folgenden Schritte umfasst:

    - Aktivieren von zwischen 20 und 200 % in der seitlichen Richtung (Y) einer Zone einer ersten Vliesmatte (12), die sich über eine in der Längsrichtung (X) gemessene Länge der ersten Vliesmatte und über eine Breite erstreckt, die strikt kleiner als eine in der seitlichen Richtung (Y) gemessene Breite der ersten Vliesmatte ist;
    - Bereitstellen eines elastischen Films (24);
    - Laminieren der ersten Vliesmatte (12) und des elastischen Films (24),
    - Bereitstellen einer zweiten Vliesmatte (30),
    - Laminieren der zweiten Vliesmatte (30) auf die erste Vliesmatte (12),

    wobei sich die Aktivierungsrate der aktivierten Zone (14) der ersten Vliesmatte (12) in der seitlichen Richtung (Y) von der Aktivierungsrate des aktivierten Zone (14) der zweiten Vliesmatte (30) in der seitlichen Richtung (Y) unterscheidet.

11. Herstellungsverfahren nach einem der Ansprüche 9 oder 10, das vor dem Laminieren der Vliesmatte (12) einen Schritt einer Beschichtung der Vliesmatte (12) mit einem Klebstoff (18) umfasst.

12. Herstellungsverfahren nach einem der Ansprüche 9 bis 11, wobei vor dem Laminieren der zweiten Vliesmatte (30) die zweite Vliesmatte (30) zwischen 20 und 200 % in der seitlichen Richtung (Y) über eine Zone aktiviert wird, die sich über eine in der Längsrichtung (X) gemessene Länge der Vliesmatte und über eine Breite erstreckt, die strikt kleiner als eine in der seitlichen Richtung (Y) gemessene Breite der Vliesmatte ist.

13. Herstellungsverfahren nach einem der Ansprüche 9 bis 12, das vor dem Laminieren der zweiten Vliesmatte (30) einen Schritt eines Beschichtens der zweiten Vliesmatte (30) mit einem Klebstoff (18) umfasst.

14. Herstellungsverfahren nach einem der Ansprüche 9 bis 13, wobei das Laminat (10) mindestens zu 200 % in der seitlichen Richtung (Y) aktiviert wird.

Claims

1. A laminated assembly (10) extending in a longitudinal direction (X) and a lateral direction (Y) orthogonal to the longitudinal direction (X), the assembly comprising a first non-woven sheet (12) and an elastic film (24) that are laminated together, the first non-woven sheet (12) including at least one activated zone (14) extending over the length of the non-woven sheet measured in the longitudinal direction (X) and over a width that is strictly less than the width of the non-woven sheet measured in the lateral direction (Y), the degree of activation of the activated zone (14) of the first non-woven sheet (12) in the lateral direction (Y) being different from the degree of activation of the elastic film (24) in the lateral direction (Y), the degree of activation of the activated zone (14) of the first non-woven sheet (12) in the lateral direction (Y) lying in the range 20% to 200%, the laminated assembly (10) including a second non-woven sheet (30), the elastic film (24) being interposed between the first and second non-woven sheets (12, 30),

wherein the degree of activation of the activated zone (14) of the first non-woven sheet (12) in the lateral direction (Y) is different from the degree of activation of the activated zone (14) of the second non-woven sheet (30) in the lateral direction (Y).

2. A laminated assembly (10) extending in a longitudinal direction (X) and a lateral direction (Y) orthogonal to the longitudinal direction (X), the assembly comprising a first non-woven sheet (12) and an elastic film (24) that are laminated together, the first non-woven sheet (12) including at least one activated zone (14) extending over the length of the non-woven sheet measured in the longitudinal direction (X) and over a width that is strictly less than the width of the non-woven sheet measured in the lateral direction (Y), the degree of activation of the activated zone (14) of the first non-woven sheet (12) in the lateral direction (Y) being different from the degree of activation of the elastic film (24) in the lateral direction (Y), the degree of activation of the activated zone (14) of the first non-woven sheet (12) in the lateral direction (Y) lying in the range 20% to 200%, the laminated assembly (10) including a second non-woven sheet (30) and the second non-woven sheet (30) being interposed at least in part between the first non-woven sheet (12) and the elastic film (24),

wherein the degree of activation of the activated zone (14) of the first non-woven sheet (12) in the lateral direction (Y) is different from the degree of activation of the activated zone (14) of the second non-woven sheet (30) in the lateral direction (Y).

3. The laminated assembly (10) according to one of claims 1 or 2, wherein the second non-woven sheet (30) does not include an activated zone.

4. The laminated assembly (10) according to one of claims 1 or 2, wherein the second non-woven sheet (30) includes at least one activated zone (14) extending over the length of the non-woven sheet measured in the longitudinal direction (X) and over a width that is strictly less than the width of the non-woven sheet measured in the lateral direction (Y), the degree of activation of the activated zone (14) of the second non-woven sheet (30) in the lateral direction (Y) lying in the range 20% to 200%.

5. The laminated assembly (10) according to any one of claims 1 to 4, wherein the degree of activation of the activated zone (14) of the second non-woven sheet (30) in the lateral direction (Y) is different from the degree of activation of the elastic film (24) in the lateral direction (Y).

6. The laminated assembly (10) according to any one of claims 1 to 5, wherein the non-woven sheet (12, 30) is a carded and calendared non-woven fabric.

7. The laminated assembly (10) according to any one of claims 1 to 5, wherein the non-woven sheet (12, 30) is a non-woven fabric of Spunlace type.

8. The laminated assembly (10) according to any one of claims 1 to 5, wherein the non-woven sheet (12, 30) is a non-woven fabric of Spunmelt type.

9. A fabrication method for fabricating a laminated assembly (10) extending in a longitudinal direction (X) and in a lateral direction (Y) orthogonal to the longitudinal direction (X), the method comprising the following steps:

   - activating in the lateral direction (Y) to a degree in the range 20% to 200% a zone of a first non-woven sheet (12) extending over a length of the first non-woven sheet measured in the longitudinal direction (X) and over a width that is strictly less than the width of the first non-woven sheet measured in the lateral direction (Y);
   - providing an elastic film (24);
   - laminating the first non-woven sheet (12) with the elastic film (24),
   - providing a second non-woven sheet (30),
   - laminating the second non-woven sheet (30) on the elastic film (24)

wherein the degree of activation of the activated zone (14) of the first non-woven sheet (12) in the lateral direction (Y) is different from the degree of activation of the activated zone (14) of the second non-woven sheet (30) in the lateral direction (Y).

10. A fabrication method for fabricating a laminated assembly (10) extending in a longitudinal direction (X) and in a lateral direction (Y) orthogonal to the longitudinal direction (X), the method comprising the following steps:

  - activating in the lateral direction (Y) to a degree in the range 20% to 200% a zone of a first non-woven sheet (12) extending over a length of the first non-woven sheet measured in the longitudinal direction (X) and over a width that is strictly less than the width of the first non-woven sheet measured in the lateral direction (Y);
  - providing an elastic film (24);
  - laminating the first non-woven sheet (12) with the elastic film (24),
  - providing a second non-woven sheet (30),
  - laminating the second non-woven sheet (30) on the first non-woven sheet (12)

  wherein the degree of activation of the activated zone (14) of the first non-woven sheet (12) in the lateral direction (Y) is different from the degree of activation of the activated zone (14) of the second non-woven sheet (30) in the lateral direction (Y).

11. The fabrication method according to one of claims 9 or 10, including, prior to laminating the non-woven sheet (12), a step of coating the non-woven sheet (12) with an adhesive (18).

12. The fabrication method according to one of claims 9 to 11, wherein, prior to laminating the second non-woven sheet (30), the second non-woven sheet (30) is activated in the lateral direction (Y) to a degree lying in the range 20% to 200% over a zone extending over the length of the non-woven sheet measured in the longitudinal direction (X) and over a width that is strictly less than the width of the non-woven sheet measured in the lateral direction (Y).

13. The fabrication method according to one of claims 11 to 12, including, prior to laminating the second non-woven sheet (30), a step of coating the second non-woven sheet (30) with an adhesive (18).

14. The fabrication method according to any one of claims 9 to 13, wherein the laminated assembly (10) is activated to a degree of less than 200% in the lateral direction (Y).

FIG.1A

FIG.1B

FIG.2

FIG.3A

FIG.3B

FIG.3C

FIG.4

FIG.5

FIG.6A

FIG.6B

FIG.7

FIG.8

FIG.9

FIG.10

24

**EP 3 484 702 B2**

## RÉFÉRENCES CITÉES DANS LA DESCRIPTION

**Documents brevets cités dans la description**

- EP 0803602 A